# EUROPEAN PATENT APPLICATION

(11) **EP 1 426 059 A1**
(43) Date of publication of application: **09.06.2004**
(21) Application number: 02779881.8
(22) Date of filing: 13.09.2002
(51) Int. Cl.: A61K 45/00, A61P 17/04

(54) **REMEDIES FOR PRURITUS**

(30) Priority: 14.09.2001 JP 2001280438
(71) Applicant: ONO PHARMACEUTICAL CO., LTD., Osaka-shi, Osaka 541-8526 (JP)
(72) Inventor: NARITA, Masami, c/o Minase Res. Inst., Mishima-gun, Osaka 618-8585 (JP); SATO, Kazutoyo, c/o Minase Res. Inst., Mishima-gun,, Osaka 618-8585 (JP); KOBAYASHI, Kaoru, c/o Minase Res. Inst., Mishima-guun, Osaka 618-8585 (JP)
(74) Representative: Henkel, Feiler & Hänzel
(86) International application number: PCT/JP2002/009439
(87) International publication number: WO 2003/024484

(57) **Abstract**

The present invention relates to agents for treating and/or preventing pruritus which comprise, as the active ingredient, the compound with antagonistic activity for EP₃ receptor which is one of prostaglandin E₂ receptor subtypes. The compounds with antagonistic activity for EP₃ are useful in treating and/or preventing pruritus in diseases with itch, example for, eczema, urticaria, contact dermatitis, atopic dermatitis, dermatitis herpetiformis, psoriasis, lichen planus, rhus dermatitis, biliary obstruction, uremia, lymphoma, leukemia, polycythemia vera, dry skin, or hemodialysis performed in treating renal involvement with chronic glomerulonephritis, diabetes mellitus, nephrosclerosis, cystic kidney or systemic disease, or conjunctivitis.

## Description

### Technical Field

The present invention relates to therapeutic agents for pruritus. More specifically, it relates to agents for treating and/or preventing pruritus which comprise, as the active ingredient, the compound with antagonistic activity for EP₃ which is one of prostaglandin E₂ receptor subtypes.

### Background

Prostaglandin E₂ (abbreviated as PGE₂) has been known as a metabolite in the arachidonic acid cascade. It has been known that PGE₂ possesses cyto-protective activity, uterine contractile activity, a pain-inducing effect, a promoting effect on digestive peristalsis, an awaking effect, a suppressive effect on gastric acid secretion, hypotensive activity, and diuretic activity *etc*.

In the recent study, it was found hat PGE₂ receptor was divided into some subtypes which possesses different physical roles from each other. At present, four receptor subtypes are known and they are called EP₁, EP₂, EP₃ and EP₄, respectively [*J. Lipd Mediators Cell Signaling*, 12, 379-391 (1995)].

Among these subtypes, EP₃ receptor was revealed to be involved in signal transduction of peripheral nerve, control of exothermal reaction in central nerve, formation of memory by expressing in cerebral neuron, vascularization, reabsorption of urine by expressing in renal tubular, uterine contraction, production of ACTH, platelet aggregation. Besides, it was expressed in vascular smooth muscle, heart and gastrointestinal tract also.

However, it is not shown clearly whether the compound with antagonistic activity for EP₃ receptor is concretely used for treatment of what disease.

On the other hand, pruritus is induced by various diseases. As skin diseases inducing pruritus, eczema, urticaria, contact dermatitis, atopic dermatitis, dermatitis herpetiformis, psoriasis, lichen planus, rhus dermatitis *etc*. are given. As diseases without skin lesions, biliary obstruction, uremia, lymphoma, leukemia, and polycythemia vera *etc*. are given. Dry skin causes systemic pruritus. Moreover, pruritus is caused by hemodialysis performed in treating renal involvement with chronic glomerulonephritis, diabetes mellitus, nephrosclerosis, cystic kidney or systemic disease, or conjunctivitis, for example, seasonal allergic conjunctivitis, acute conjunctivitis, chronic conjunctivitis, trachoma, perennial allergic conjunctivitis or spring.

As substances inducing these pruritus, histamine, serotonin, substance-P or leukotriene B4 etc. are nominated, and antihistamines, antiserotonins, antiallergic agents or steroids etc. are used for patients. However, these effects are not yet enough and there is also a problem of side effects.

In the specification of WO01/62708, it is described that the compounds represented by formula (A) 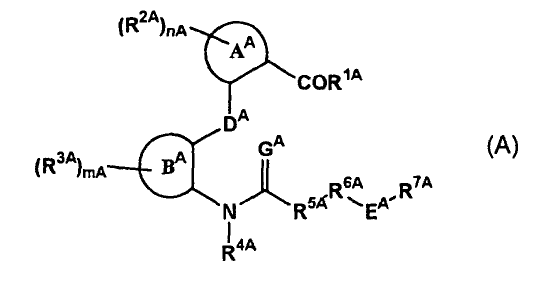 (wherein, all symbols have the same meanings as defined hereinafter.) strongly bind to and antagonize PGE₂ receptor, particularly the subtype EP₄ and the compounds are useful in preventing and/or treating bone diseases (osteoporosis, rheumatoid arthritis, osteoarthritis or abnormal bone formation *etc*.), cancer (formation, proliferation, metastasis to organs, bone metastasis or hypercalcemia that accompanies bone metastasis *etc*.), systemic granuloma, immunological diseases (amyotrophic lateral sclerosis (ALS), multiple sclerosis, Sjoegren's syndrome, systemic lupus erythematosus or AIDS *etc.*), allergy (conjunctivitis, rhinitis, contact dermatitis or psoriasis *etc.*), atopy (atopic dermatitis *etc.*), asthma, pyorrhea, gingivitis, periodontitis, neuronal cell death, Alzheimer's disease, pulmonary injury, hepatopathy, acute hepatopathy, nephritis, renal failure, myocardiac ischemia, Kawasaki disease, scald, ulcerative colitis, Crohn's disease, multiple organ failure, sleeping disorder or platelet aggregation etc.

In the specification, it is not described that the compounds represented by formula (A) have EP₃ antagonistic action. Also, it is not described with respect to pruritus. Therefore, the relation between EP₃ antagonism and pruritus cannot be predicted.

It is described that the compounds represented by formula (B) 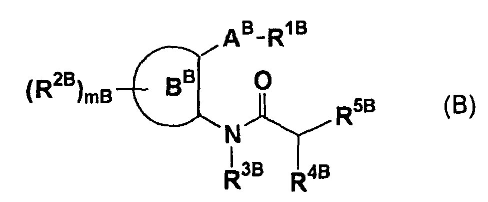 (wherein, all symbols have the same meanings as defined hereinafter.) in the specification of WO02/16311, the compounds represented by formula (C) 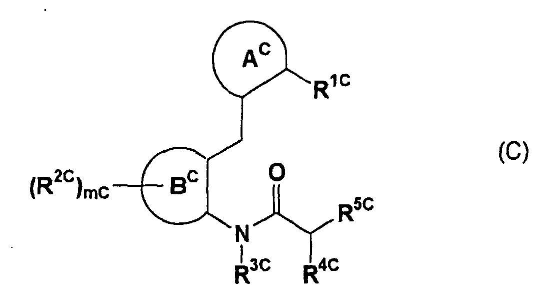 (wherein, all symbols have the same meanings as defined hereinafter.) in the specification of WO02/20462, and the compounds represented by formula (D) 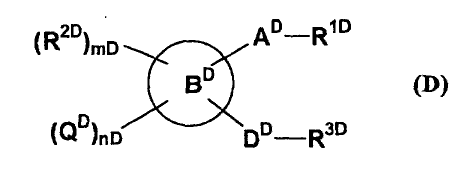 (wherein, all symbols have the same meanings as defined hereinafter.) in the specification of PCT/JP02/08120 strongly bind to and antagonize PGE₂ receptor, particularly the subtype EP₃ and/or EP₄ and the compounds are useful in preventing and/or treating pain such as cancerous pain, fractural pain, pain following surgical and dental procedures; allodynia, hyperalgesia, pruritus, urticaria, atopic dermatitis, contact dermatitis, rhus dermatitis, allergic conjunctivitis, various symptoms by treating with dialysis, asthma, rhinitis, sneeze, urinary frequency such as neurogenic bladder, neurogenic bladder, irritant bladder, unstable bladder, urinary frequency that originate with prostate-gland enlargement; urinary disturbance, ejaculatory failure, fever, systemic inflammatory response syndrome, learning disturbance, Alzheimer's disease, angiogenesis, cancer such as formulation of cancer, growth of cancer and metastasis of cancer; retinopathy, patch of red, erythematous patches, achromoderma, pigmented spot, scald, burn, burn by steroid, renal failure, nephropathy, acute nephritis, chronic nephritis, abnormal blood levels of electrolytes, threatened premature delivery, abortion threatened, hypermenorrhea, dysmenorrhea, uterine fibroids, premenstrual syndrome, reproductive disorder, stress, anxiety disorders, depression, psychosomatic disorder, mental disorder, thrombosis, embolism, transient ischemia attack, cerebral infarction, atheroma, organ transplant, myocardial infarction, cardiac failure, hypertension, arteriosclerosis, circulatory failure and circulatory failure induced ulcer, neuropathies, vascular dementia, edema, various arthritis, rheumatism, diarrhea, constipation, disorder of bilious excretion, ulcerative colitis, Crohn's disease, irritable bowel syndrome, alleviation of rebound phenomenon after steroid, dose reduction of steroid and adjunct for steroid withdrawal and/or bone diseases such as osteoporosis, rheumatoid arthritis, osteoarthritis, abnormal bone formation; cancer such as formation of cancer, proliferation of cancer, metastasis of cancer to organs and to bones and hypercalcemia induced metastasis to bones of cancer; systemic granuloma, immunological diseases such as ALS, multiple sclerosis, Sjoegren's syndrome, systemic lupus erythematosus, AIDS; allergy such as allergic conjunctivitis, allergic rhinitis, contact dermatitis, psoriasis; atopy such as atopic dermatitis; asthma, pyorrhea, gingivitis, periodontitis, neuronal cell death, Alzheimer's disease, pulmonary injury, hepatopathy, acute hepatopathy, nephritis, renal failure, myocardial ischemia, Kawasaki disease, scald, ulcerative colitis, Crohn's disease, multiple organ failure, chronic headache such as migraine headache, tension-type headache or mixed headache thereof, cluster headache; pain, angiogenesis, angiitis, venous insufficiency, varicose veins, anal fistula, diabetes insipidus, stress, endometriosis, adenomyosis of the uterus, neonatal patent ductus arteriosus, cholelithiasis *etc*.

In these specifications, the relation between EP₃ and/or EP₄ antagonism and pruritus, urtication, atopic dermatitis, contact dermatitis or various symptoms in dialysis is indicated. However, a concrete experiment and proof are not carried out.

It is described that the compounds represented by formula (E) 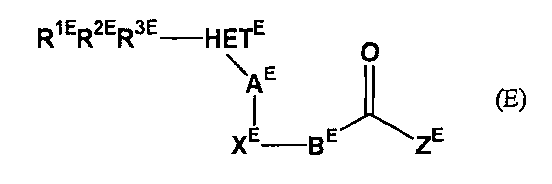 (wherein, all symbols have the same meanings as defined hereinafter.) in the specification of WO99/47497, the compounds represented by formula (F)

Ar^{1F}-W^{F}-Ar^{2F}-X^{F}-Q^{F} (F)

(wherein, all symbols have the same meanings as defined hereinafter.) in the specification of WO00/20371, the compounds represented by formula (G) 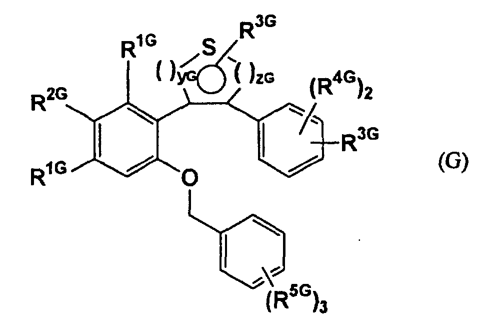 (wherein, all symbols have the same meanings as defined hereinafter.) in the specification of WO2001/19814 and the compounds represented by formula (H) 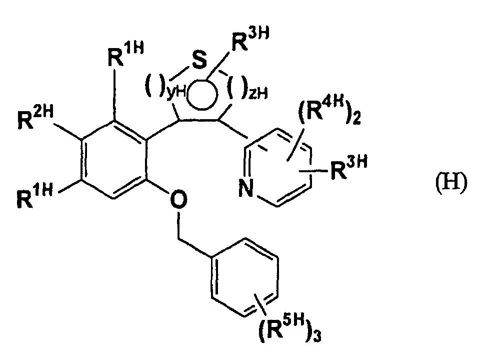 (wherein, all symbols have the same meanings as defined hereinafter.) in the specification of WO2001/19819 are useful for treatment and/or prevention of the diseases caused by prostaglandin such as pain, fever or inflammation associated with rheumatic fever, influenza or other viral infections, common cold, low back and neck pain, skeletal pain, post-partum pain, dysmenorrhea, headache, migraine, toothache, sprains and strains, myositis, neuralgia, synovitis, arthritis, including rheumatoid arthritis, degenerative joint diseases (osteoarthritis), gout and ankylosing spondylitis, bursitis, burns including radiation and corrosive chemical injuries, sunburns, pain following surgical and dental procedures, immune and autoimmune diseases, cellular neoplastic transformations or metastic tumor growth, diabetic retinopathy, tumor angiogenesis, prostanoid-induced smooth muscle contraction associated with dysmenorrhea, premature labor, asthma or eosinophil related disorders, Alzheimer's disease, glaucoma, bone loss, osteoporosis, promotion of bone formation, Paget's disease, cytoprotection in peptic ulcers, gastritis, regional enteritis, ulcerative colitis, diverticulitis or other gastrointestinal lesions, GI bleeding and patients undergoing chemotherapy, coagulation disorders selected from hypoprothrombinemia, haemophilia and other bleeding problems, kidney disease, thrombosis, occlusive vascular disease, presurgery and anticoagulation.

However, the relation between PGE₂ receptor subtypes and the specific disease is not shown in these specifications. Moreover, the relation between EP₃ antagonism and pruritus is not shown and therefore it cannot be predicted that the compounds which have EP₃ antagonistic activity are effective in pruritus.

### Disclosure of the invention

PGE₂ receptor was divided into four subtypes, which possesses different physical roles from each other. Among those, the concrete indication in which the compound which has antagonism to EP₃ receptor is effective is not y*etc*larified and the medicine which comprises the compound having antagonistic activity for EP₃ receptor as an active ingredient to is not realized.

The present inventors have energetically studied to find the compounds which are useful for treating and/or preventing pruritus. As a result, they found out that the compounds which have EP₃ receptor antagonistic activity achieve the purpose. Moreover, they found out that the compounds described in the specifications of WO01/62708, WO99/47497, WO00/20371, WO2001/19814 and WO2001/19819 which were not known to have antagonism to EP₃ receptor, have antagonism to EP₃ receptor. Accordingly they found out that the purpose was achieved, and the present invention has been accomplished.

The present invention is relates to agents for treating and/or preventing pruritus which comprise, as the active ingredient, the compound with antagonistic activity for EP₃ which is one of prostaglandin E₂ receptor subtypes.

All the compounds that have antagonism to EP₃ receptor among the compounds known by present as a compound which has antagonism to EP₃ receptor used for the present invention are included. Preferably, the following compounds are used.
(1) In the specification of WO01/62708, a benzoic acid derivative represented by formula (A) 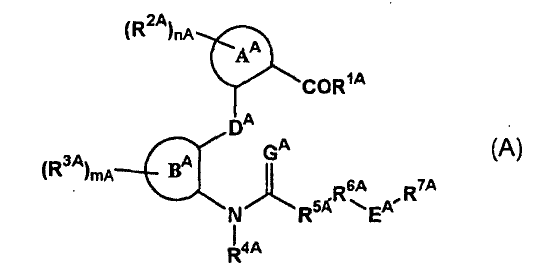 (wherein 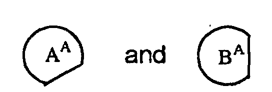 are each independently C3-7 carbocyclic ring or 5-7 membered heterocyclic ring containing nitrogen, sulfur and/or oxygen atom,
   D^{A} is C1-4 alkylene, oxygen or sulfur atom,
   G^{A} is oxygen or sulfur atom,
   E^{A} is a bond, oxygen, sulfur, C1-4 alkylene, C1-4 alkyloxy or C1-4 oxyalkyl,
   R^{1A} is hydroxyl, -OR^{9A} or -NR^{10A}R^{11A},
   (wherein R^{9A} is C1-6 alkyl, R^{10A} and R^{11A} are each independently, hydrogen atom or C1-6 alkyl.),
   R^{2A} and R^{3A} are each independently, C1-4 alkyl, C1-4 alkoxy, halogen atom, trihalomethyl, cyano or nitro,
   R^{4A} is hydrogen atom or C1-6 alkyl,
   R^{5A} is a bond, C1-6 alkylene, C1-6 alkylene substituted with C1-4 alkoxy, or C3-5 cycloalkylene,
   R^{6A} is C5-15 carbocyclic ring or 5-15 membered heterocyclic ring containing nitrogen, sulfur and/or oxygen atom,
   R^{7A} is hydrogen, C1-8 alkyl, C5-7 carbocyclic ring or 5-15 membered heterocyclic ring containing nitrogen, sulfur and/or oxygen atom,
   m^{A} and n^{A} are each independently, 0, 1, 2 or 3,
   the rings represented by R^{6A} and R^{7A} may be substituted with C1-4 alkyl, C1-4 alkoxy, halogen atom, trihalomethyl, nitro, cyano or oxom, wherein 2-[2-(benzoylamino)phenylmethyl]benzoic acid is excluded.) or a non-toxic salt thereof.
(2) In the specification of W002/16311, a carboxylic acid derivative represented by formula (B) 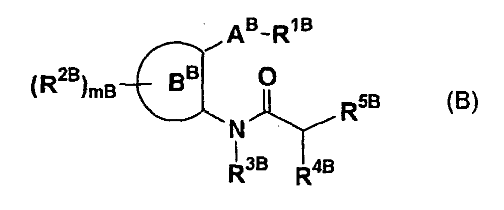 (wherein R^{1B} is COOH, COOR^{6B}, CH₂OH, CONHSO₂R^{7B} or CONR^{8B}R^{9B},
   R^{6B} is C1-6 alkyl, (C1-4 alkylene)-R^{16B},
   R^{7B} is (1) C1-4 alkyl, or (2) (2-1) C6-12 mono- or bi-carbocyclic ring or (2-2) 5-15 membered mono- or bi-heterocyclic ring containing at least one of hetero atom selected from nitrogen, oxygen and sulfur atom substituted with 1-2 of substitutes selected form C1-4 alkyl, C1-4 alkoxy and halogen atom or unsubstituted, or (3) C1-4 alkyl substituted with the above carbocyclic ring or heterocyclic ring substituted or unsubstituted,
   R^{8B} and R^{9B} are each independently hydrogen or C1-4 alkyl,
   R^{16B} is hydroxy, C1-4 alkoxy, COOH, C1-4 alkoxycarbonyl or CONR^{8B}R^{9B},
   A^{B} is C1-6 alkylene or -(C1-3 alkylene)_{WB}-G^{B}-(C1-3 alkylene)-,
   W^{B} is 0 or 1,
   G^{B} is oxygen atom, sulfur atom or NR^{10B},
   R^{10B} is hydrogen atom or C1-4 alkyl,
   R^{2B} is C1-6 alkyl, C2-6 alkenyl, C2-6 alkynyl, C1-6 alkoxy, halogen atom, CF₃, cyano, nitro, hydroxy, NR^{11B}R^{12B}, CONR^{11B}R^{12B}, SO₂R^{11B}R^{12B} or -S(O)_{XB}-(C1-6) alkyl,
   m^{B} is 0, 1 or 2, and when m^{B} is 2, then two R^{2B} may be same or difference,
   R^{11B} and R^{12B} are each independently, hydrogen or C1-4 alkyl,
   X^{B} is 0, 1 or 2,
   B^{B} ring is C5-7 mono-carbocyclic ring or 5-7 membered mono-heterocyclic ring containing at least one of nitrogen, oxygen and sulfur atom,
   R^{3B} is hydrogen atom or C1-4 alkyl,
   R^{4B} is (1) C1-8 alkyl, (2) C2-8 alkenyl, (3) C2-8 alkynyl, (4) C3-6 cycloalkyl, (5) hydroxy, (6) C1-4 alkoxy, (7) C1-4 alkoxy(C1-4)alkoxy, or (8) C-8 alkyl substituted with 1-2 of substitutes selected from halogen atom, hydroxy, C1-6 alkoxy, C1-4 alkoxy(C1-4)alkoxy, phenyl and C3-6 cycloalkyl,
   R^{5B} is C5-10 mono- or bi-carbocyclic ring or 5-10 membered mono- or bi-heterocyclic ring containing at least one of nitrogen, oxygen and sulfur atom substituted with 1-2 of R^{13B} or unsubstituted,
   R^{13B} is C1-6 alkyl, C1-6 alkoxy, halogen atom, CF₃, cyano, C1-4 alkoxy(C1-4)alkyl, phenyl, phenyl(C1-6)alkyl, -(C1-4 alkylene)_{YB}-J^{B}-(C1-8 alkylene)_{ZB}-R^{14B}, benzoyl or thiophenecarbonyl and when two R^{13B} exist, they may be same or difference,
   Y^{B} is 0 or 1,
   Z^{B} is 0 or 1,
   R^{14B} is phenyl or pyridyl,
   J^{B} is oxygen atom, S(O)_{tB}, NR^{15B},
   t^{B} is 0, 1 or 2,
   R^{15B} is hydrogen atom, C1-4 alkyl or acetyl.) or a non-toxic salt thereof.
(3) In the specification of W002/20462, a benzoic acid derivative represented by formula (C) 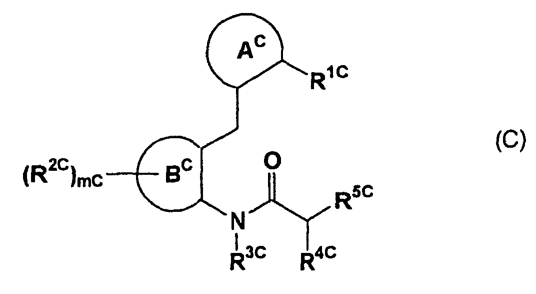 (wherein R^{1C} is COOH, COOR^{6C}, CH₂OH, CONHSO₂R^{7C} or CONR^{8C}R^{9C},
   R^{6C} is C1-6 alkyl or (C1-4 alkylene)-R^{16C},
   R^{7C} is (1) C1-4 alkyl or (2) (2-1) C6-12 mono- or bi-carbocyclic ring or (2-2) 5-15 membered mono- or bi-heterocyclic ring containing at least one of hetero atom selected from nitrogen atom, oxygen atom and sulfur atom substituted by 1-2 of substitutes selected form C1-4 alkyl, C1-4 alkoxy and halogen atom or unsubstituted, or (3) the above C1-4 alkyl substituted by carbocyclic ring or heterocyclic ring substituted or unsubstituted,
   R^{8C} and R^{9C} are each independently, hydrogen atom or C1-4 alkyl,
   R^{16C} is hydroxy, C1-4 alkoxy, COOH, C1-4 alkoxycarbonyl, CONR^{8C}R^{9C},
   A^{C} is C5-6 mono-carbocyclic ring or 5-6 membered mono-heterocyclic ring containing at least one of nitrogen atom, oxygen atom and sulfur atom, and the mono-carbocyclic ring or mono-heterocyclic ring may be substituted by 1-2 of substitutes selected from C1-4 alkyl, C1-4 alkoxy, halogen atom, CF₃, cyano and nitro,
   R^{2C} is C1-6 alkyl, C2-6 alkenyl, C2-6 alkynyl, C1-6 alkoxy, halogen atom, CF₃, cyano, hydroxy, nitro, NR^{10C}R^{11C}, CONR^{10C}R^{11C}, -SO₂NR^{10C}R^{11C} or -S(O)_{XC}-C1-4 alkyl,
   m^{C} is 0, 1 or 2, and when m^{C} is 2, then two R^{2C} may be same or difference,
   R^{10C} and R^{11C} are each independently, hydrogen atom or C1-4 alkyl,
   X^{C} is 0, 1 or 2,
   B^{C} is C5-7 mono-carbocyclic ring or 5-7 membered mono-heterocyclic ring containing at least one of nitrogen atom, oxygen atom and sulfur atom,
   R^{3C} is hydrogen atom or C1-4 alkyl,
   R^{4C} is (1) C1-8 alkyl, (2) C2-8 alkenyl, (3) C2-8 alkynyl, (4) C3-6 cycloalkyl, (5) hydroxy, (6) C1-6 alkoxy, (7) C1-4 alkoxy(C1-4)alkoxy, or (8) C1-8 alkyl substituted with 1-2 of substitutes selected from halogen atom, hydroxy, C1-6 alkoxy, C1-4 alkoxy(C1-4)alkoxy, phenyl and C3-6 cycloalkyl, R^{5C} is C5-10 mono- or bi-carbocyclic ring or 5-10 membered mono- or bi-heterocyclic ring containing at least one of nitrogen atom, oxygen atom and sulfur atom substituted with 1-2 of R^{12C} or unsubstituted,
   R^{12C} is C1-6 alkyl, C1-6 alkoxy, halogen atom, CF₃, cyano, C1-4 alkoxy(C1-4)alkyl, phenyl, phenyl(C1-6)alkyl, -(C1-4 alkylene)_{YC}-G^{C}-(C1-8 alkylene)_{ZC}-R^{13C}, benzoyl or thiophenecarbonyl and two when R^{12C} exist, they may be same or difference,
   Y^{C} is 0 or 1,
   Z^{C} is 0 or 1,
   R^{13C} is phenyl or pyridyl,
   G^{C} is oxygen atom, S(O)_{tC} or NR^{14C},
   t^{C} is 0, 1 or 2,
   R^{14C} is hydrogen atom, C1-4 alkyl or acetyl.) or a non-toxic salt thereof.
(4) In the specification of PCT/JP02/08120, a carboxylic acid derivative represented by formula (D) 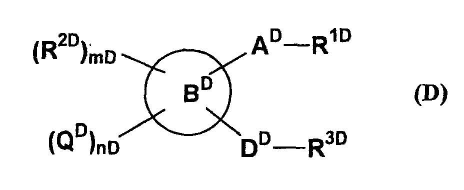 (wherein R^{1D} is -COOH, -COOR^{4D}, -CH₂-OH, -CONR^{5D}SO₂R^{6D}, -CONR^{7D}R^{8D}, -CH₂-NR^{5D}SO₂R^{6D}, -CH₂-NR^{9D}COR^{10D}, -CH₂-NR^{9D}CONR^{5D}SO₂R^{6D}, -CH₂-SO₂NR^{9D}COR^{10D}, -CH₂-OCONR^{5D}SO₂R^{6D}, tetrazole, 1,2,4-oxadiazol-5-one, 1,2,4-oxadiazol-5-tione, 1,2,4-thiadiazol-5-one, 1,3-thiazolidin-2,4-dione or 1,2,3,5-oxathiadiazol-2-one,
   R^{4D} is C1-6 alkyl or -(C1-4 alkylene)-R^{11D},
   R^{11D} is hydroxyl, C1-4 alkoxy, -COOH, C1-4alkoxycarbonyl or -CONR^{7D}R^{8D},
   R^{5D} is hydrogen atom or C1-6 alkyl,
   R^{6D} is, (i) C1-6 alkyl,
   (ii) C3-15 mono-, bi- or tri-carbocyclic ring or a 3- to 15-membered mono-, bi- or tri-heterocyclic ring substituted with 1-5 of R^{12D} or unsubstituted,
   (iii) C1-6 alkyl, C2-6 alkenyl or C2-6 alkynyl substituted with a C3-15 mono-, bi- or tri-carbocyclic ring or a 3- to 15-membered mono-, bi- or tri-heterocyclic ring substituted with 1-5 of R^{12D} or unsubstituted,
      R^{7D} and R^{8D} each independently, are (i) hydrogen atom,
   (ii) C1-6 alkyl,
   (iii) hydroxy,
   (iv) -COR^{17D},
   (v) a C3-15 mono-, bi- or tri-carbocyclic ring or a 3- to 15-membered mono-, bi- or tri-heterocyclic ring substituted with 1-5 of R^{12D} or unsubstituted, or
   (vi) C1-4 alkyl substituted with a C3-15 mono-, bi- or tri-carbocyclic ring or a 3- to 15-membered mono-, bi- or tri-heterocyclic ring which is substituted with 1-5 of R^{12D} or unsubstituted,
      R^{9D} is hydrogen atom or C1-6 alkyl,
      R^{10D} is, (i) hydrogen atom,
   (ii) C1-6 alkyl,
   (iii) a C3-15 mono-, bi- or tri-carbocyclic ring or a 3- to 15-membered mono-, bi- or tri-heterocyclic ring substituted with 1-5 of R^{12D} or unsubstituted, or
   (iv) C1-6 alkyl, C2-6 alkenyl or C2-6 alkynyl substituted with a C3-15 mono-, bi- or tri-carbocyclic ring or a 3- to 15-membered mono-, bi- or tri-heterocyclic ring substituted with 1-5 of R^{12D} or unsubstituted,
      R^{12D} is (a) C1-6 alkyl, (b) C1-6 alkoxy, (c) C1-6 alkylthio, (d) halogen atom, (e) CF₃, (f) cyano, (g) nitro, (h) hydroxy, (i) -COOR^{13D}, (j) -NHCOR^{13D}, (k) -SO₂R^{14D}, (l) -NR^{15D}R^{16D}, (m) a C3-7 mono-carbocyclic ring substituted with C1-4 alkyl or oxo or unsubstituted, (n) a 3- to 7-membered mono-heterocyclic ring substituted with C1-4 alkyl or oxo or unsubstituted, or (o) C1-4 alkyl substituted with hydroxy, -COOR^{13D}, -NHCOR^{13D}, -SO₂R^{14D} or -NR^{15D}R^{16D},
      R^{13D} is hydrogen, C1-4 alkyl, phenyl, or phenyl(C1-4) alkyl,
      R^{14D} is C1-4 alkyl,
      R^{15D} and R^{16D} are each independently, hydrogen atom, C1-4 alkyl, phenyl, phenyl(C1-4) alkyl,
      R^{17D} is C1-4 alkyl or phenyl,
      A^{D} is, (i) a single bond,
      (ii) C1-6 alkylene,
      (iii) C2-6 alkenylene,
      (iv) C2-6 alkynylene,
      (v) -O-(C1-3 alkylene),
      (vi) -S-(C1-3 alkylene),
      (vii) -NR^{20D}-(C1-3 alkylene),
      (viii) -CONR^{21D}-(C1-3 alkylene),
      (ix) -(C1-3 alkylene)-O-(C1-3 alkylene),
      (x) -(C1-3 alkylene)-S-(C1-3 alkylene),
      (xi) -(C1-3 alkylene)-NR^{20D}-(C1-3 alkylene),
      (xii) -(C1-3 alkylene)-CONR^{21D}-(C1-3 alkylene),
      (xiii) -Cyc1^{D},
      (xiv) -(C1-4 alkylene)-Cyc1^{D}, or
      (xv) -Cyc1^{D}-(C1-4 alkylene),
the alkylene, alkenylene and alkynylene in A^{D} may be substituted with 1-6 of the following substituents of (a)-(i):
(a) C1-6 alky, (b) C1-6 alkoxy, (c) halogen atom, (d) CHF₂, (e) CF₃,
(f) OCHF₂, (g) OCF₃, (h) hydroxy, (i) hydroxy(C1-4) alkyl,
   R^{20D} is hydrogen atom, C1-4 alkyl, -SO₂(C1-4)alkyl or C2-5 acyl,
   R^{21D} is hydrogen atom or C1-4 alkyl,
   Cyc1^{D} is a C3-7 mono-carbocyclic ring or a 3- to 7-membered mono-heterocyclic ring substituted with 1-4 of C1-6 alkyl, C1-6 alkoxy, C1-6alkylthio, C2-6 alkenyl, C2-6 alkynyl, halogen atom, CHF₂, CF₃, nitro and cyano or unsubstituted,
B^{D} ring is a C3-12 mono- or bi-carbocyclic ring or a 3- to 12-membered mono- or bi-heterocyclic ring,
R^{2D} is C1-6 alkyl, C1-6 alkoxy, C1-6 alkylthio, C2-6 alkenyl, C2-6 alkynyl, halogen atom, CHF₂, CF₃, nitro, cyano, phenyl or oxo,
m^{D} is 0, 1 or 2,
when -D^{D}-R^{3D} binds to B^{D} ring at the ortho position based on -A^{D}-R^{1D}, then n^{D} is 1 or 2,
when -D^{D}-R^{3D} binds to B^{D} ring at the non-ortho position based on -A^{D}-R^{1D}, then n^{D} is 0, 1 or 2,
Q^{D} is
(1)
   (i) -(C1-4 alkylene, C2-4 alkenylene or C2-4 alkynylene)-Cyc2^{D},
   (ii) -(C1-4 alkylene)-Z^{D}-Cyc3^{D},
   (iii) C1-4 alkyl substituted with a substituent(s) selected from -NR^{24D}R^{25D}, -S(O)_{pD}R^{26D}, cyano, -NR^{23D}COR^{27D}, -NR^{23D}SO₂R^{28D} and -NR^{23D}CONR^{24D}R^{25D} (iv) a group selected from C1-4 alkoxy(C1-4)alkoxy, -NR^{23D}COR^{27D}, -COR^{28D}, -OSO₂R^{28D}, -NR^{23D}SO₂R^{28D} and -NR^{23D}CONR^{24D}R^{25D},
   (v) a C3-7 mono-carbocyclic ring or a 3- to 6-membered mono-heterocyclic ring substituted with 1-5 of R^{30D}, wherein one R^{30D} of them binds to the ring at the non 1-position,
   (vi) a C8-15 mono-, bi- or tri-carbocyclic ring or a 7- to 15-membered mono-, bi- or tri-heterocyclic ring substituted with 1-5 of R^{30D} or unsubstituted,
   (vii) -T^{D}-Cyc5^{D},
   (viii) a group selected from -L^{D}-Cyc6^{D}-1, -L^{D}-(C3-6 cycloalkyl), -L^{D}-CH₂-(C3-6 cycloalkyl), -L^{D}-(C2-4 alkylene)-Cyc6^{D}-2 and -L^{D}-(C1-4 alkylene)_{qD}-Cyc6^{D}-3 (wherein the C3-6 cycloalkyl is substituted with 1-5 of R^{30D} or unsubstituted),
(2)
   (i) phenoxy,
   (ii) benzyloxy,
   (iii) hydroxy(C1-4)alkyl,
   (iv) C1-4alkoxy(C1-4) alkyl, or
   (v)-(C1-4 alkylene)-O-benzyl, or
(3)
   (i) C2-6 alkenyl,
   (ii) C2-6 alkynyl,
   (iii) C1-6 alkyl substituted with 1-3 halogen atom(s),
   (iv) cyano,
   (v) nitro,
   (vi) -NR^{33D}R^{34D},
   (vii) -CONR^{33D}R^{34D},
   (viii) -S(O)_{pD}-(C1-4)alkynyl,
   (ix) -S(O)_{pD}-CHF₂,
   (x) -S(O)_{pD}-NR^{33D}R^{34D},
   (xi) -O-(C3-6)alkynyl,
   (xii) -O-CHF₂, or
   (xiii) C3-7cycloalkyl,
      R^{22D} is hydrogen atom, C1-4 alkyl, -SO₂-(C1-4) alkyl or C2-5 acyl,
      R^{23D} is hydrogen atom, C1-4 alkyl, phenyl or phenyl(C1-4)alkyl,
      R^{24D} and R^{25D} are each independently, hydrogen atom or C1-4 alkylCyc4^{D} or (C1-4alkylene)-Cyc4^{D},
      R^{26D} is C1-4 alkyl or Cyc4^{D},
      R^{27D} is hydrogen atom, C1-4 alkyl or -OR^{29D} or Cyc4^{D},
      R^{28D} is C1-4 alkyl, Cyc4^{D} or -(C1-4 alkylene)-Cyc4^{D},
      R^{29D} is hydrogen atom, C1-4 alkyl, Cyc4^{D} or (C1-4 alkylene)-Cyc4^{D},
      R^{30D} is C1-8 alkyl, C1-8 alkoxy, C1-8 alkylthio, halogen atom, CF₃, OCF₃, SCF₃, CHF₂, OCHF₂, SCHF₂, hydroxy, cyano, nitro, -NR^{31D}R^{32D}, -CONR^{31D}R^{32D}, formyl, C2-5 acyl, hydroxy(C1-4)alkyl, C1-4 alkoxy(C1-4)alkyl, C1-4 alkylthio(C1-4)alkyl, -(C1-4 alkylene)-CONR^{31D}R^{32D}, -SO₂(C1-4)alkyl, -NR^{23D}CO-(C1-4)alkyl, -NR^{23D}SO₂-(C1-4)alkyl, benzoyl, oxo, a C3-7 mono-carbocyclic ring, a 3- to 7-membered mono-heterocyclic ring, -(C1-4 alkylene)-NR^{31D}R^{32D}, -M^{D}-(C3-7 mono-carbocyclic ring) or -M^{D}-(3- to 7-membered mono-heterocyclic ring),
      the C3-7 mono-carbocyclic ring and 3- to 7-membered mono-heterocyclic ring in R^{30D} may be substituted with 1-5 of the following substituents (a)-(I):
      (a) C1-6 alkyl, (b) C2-6 alkenyl, (c) C2-6 alkynyl, (d) C1-6 alkoxy, (e) C1-6 alkylthio, (f) halogen atom, (g) CHF₂, (h) CF₃, (i) nitro, (j) cyano, (k) hydroxy, (I) amino;
         M^{D} is -O-, -S-, C1-4 alkylene, -O-(C1-4 alkylene)-, -S-(C1-4 alkylene)-, -(C1-4 alkylene)-O-, or -(C1-4 alkylene)-S-,
         R^{31D} and R^{32D} are each independently, hydrogen atom or C1-4 alkyl, Cyc2^{D} is a C3-15 mono-, bi- tri-carbocyclic ring or a 3- to 15-membered mono-, bi- tri-heterocyclic ring substituted with 1-5 of R^{30D} or unsubstituted,
         Z^{D} is -O-, -S(O)_{pD}-, -NR^{22D}-, -NR^{23D}CO-, -NR^{23D}SO₂-, -NR^{22D}-(C1-4 alkylene)-, -S(O)_{pD}-(C1-4 alkylene)-, -O-(C2-4 alkylene)-, -NR^{23D}CO-(C1-4 alkylene), or -NR^{23D}SO₂-(C1-4 alkylene),
         p^{D} is 0, 1 or 2,
         Cyc3^{D} is a C3-15 mono-, bi- tri-carbocyclic ring or a 3- to 15-membered mono-, bi- tri-heterocyclic ring substituted with 1-5 of R^{30D} or unsubstituted,
         Cyc4^{D} is a C3-12 mono-, bi- tri-carbocyclic ring or a 3- to 12-membered mono-, bi- tri-heterocyclic ring substituted with 1-5 of R^{30D} or unsubstituted,
         T^{D} is -O-, -NR^{22D}-, -O-(C1-4 alkylene)-, -S(O)_{pD}-(C1-4 alkylene)-, or -NR^{22D}-(C1-4 alkylene)-,
         Cyc5^{D} is a 3- to 15-membered mono-, bi- tri-heterocyclic ring substituted with 1-5 of R^{30D} or unsubstituted,
         q^{D} is 0 or 1,
         L^{D} is -O- or -NR^{23D}-,
         Cyc6^{D}-1 is phenyl or benzyl substituted with one or more R^{30D}, Cyc6^{D}-2 is a C3-6 mono-carbocyclic ring substituted with 1-5 of R^{30D} or unsubstituted,
         Cyc6^{D}-3 is a C7-15 mono-, bi- or tri-carbocyclic ring substituted with 1-5 of R^{30D} or unsubstituted,
         R^{33D} and R^{34D} are each independently, hydrogen atom, C1-4 alkyl, phenyl or benzyl, or
         NR^{33D}R^{34D} may represent a 3- to 6-membered mono-heterocyclic ring containing one nitrogen atom and optionally containing one hetero atom selected from nitrogen, oxygen and sulfur atom,
         D^{D} is (1) a 1- or 2-membered linker comprising an atom(s) selected from carbon, nitrogen, oxygen and sulfur atom, which may contain a double bond or a triple bond and may be substituted with 1-4 of R^{40D},
(2) a 3- to 6-membered linker comprising atoms selected from carbon, nitrogen, oxygen and sulfur, which may contain a double bond or a triple bond and may be substituted with 1-12 of R^{40D}, wherein R^{40D} substituted on the atom bound to R^{3D}, and R^{42D} which is a substituent of R^{3D}, taken together may form -(CH₂)_{yD}- (in which y^{D} is 1-4.), or
(3) a 7- to 10-membered linker comprising atoms selected from carbon, nitrogen, oxygen and sulfur atom, which may contain a double bond or a triple bond and may be substituted with 1-20 of R^{40D}, wherein R^{40D} substituted on the atom bound to R^{3D}, and R^{42D} which is a substituent of R^{3D}, taken together may form -(CH₂)_{yD}-,
   R^{40D} is (a) C1-8 alkyl, (b) C2-8 alkenyl, (c) C2-8 alkynyl, (d) oxo, (e) halogen atom, (f) CF₃, (g) hydroxy, (h) C1-6 alkoxy, (i) C2-6 alkenyloxy, (j) C2-6 alkynyloxy, (k) OCF₃, (I)-S(O)_{pD}-(C1-6)alkyl, (m) -S(O)_{pD}-(C2-6)alkenyl, (n) -S(O)_{pD}-(C2-6)alkynyl, (o) C2-5 acyl, (p) Cyc9^{D}, (q) C1-4 alkoxy(C1-4)alkoxy, (r) C1-8 alkyl, C2-8 alkenyl or C2-8 alkynyl substituted with 1 or 2 of substituents selected from halogen, CF₃, OCF₃, hydroxy, C1-4 alkoxy, -S(O)_{pD}-(C1-6)alkyl, Cyc9^{D} and C1-4 alkoxy(C1-4) alkoxy, or
   two R^{40D} taken together with the atom of a linker to which they bind, may form a C3-15 mono-, bi- or tri-carbocyclic ring or a 3- to 15-membered mono-, bi- or tri-heterocyclic ring containing 1-2 hetero atom(s) selected from O, S, SO₂ and N, wherein the carbocyclic ring and the heterocyclic ring may be substituted with 1-3 substituent(s) selected from C1-4 alkyl, C1-4 alkoxy, C2-5 acyl, SO₂(C1-4 alkyl), phenyl and phenyl(C1-4) alkyl,
   Cyc9^{D} is a C3-6 mono-carbocyclic ring or a 3- to 6-membered mono-heterocyclic ring substituted with 1-5 of R^{41D} or unsubstituted,
   R^{41D} is C1-4 alkyl, C1-4 alkoxy, C1-4 alkylthio, C1-4 alkoxy(C1-4)alkyl, halogen atom, CF₃, OCF₃, SCF₃, hydroxy, cyano, formyl, C2-5 acyl, -SO₂-(C1-4)alkyl, -NR^{23D}CO-(C1-4)alkyl, benzoyl or oxo,
   R^{3D} is (1) C1-6 alkyl, or
(2) a C3-15 mono-, bi- or tri-carbocyclic ring or a 3- to 15-membered mono-, bi- or tri-heterocyclic ring substituted with 1-5 of R^{42D} or unsubstituted, R^{42D} is (a) C1-6 alkyl, (b) C1-6 alkoxy, (c) C1-6 alkylthio, (d) halogen atom, (e) cyano, (f) CF₃, (g) CHF₂, (h) OCF₃, (i) OCHF₂, (j) SCF₃, (k) -NR^{43D}R^{44D}, (I) -SO₂R^{45D}, (m) -NR^{46D}COR^{47D}, (n) hydroxy, (o) oxo, (p) C1-4 alkoxy(C1-4)alkyl, (q) Cyc10^{D}, (r) C1-6 alkylene-Cyc10^{D}, (s) -CO-Cyc10^{D}, (t) -W^{D}-Cyc10^{D}, (u) -(C1-6 alkylene)-W^{D}-Cyc10^{D}, (v) -W^{D}-(C1-6 alkylene)-Cyc10^{D}, or (w)-(C1-6 alkylene)-W^{D}-(C1-6 alkylene)-Cyc10^{D},
   R^{43D} and R^{44D} are each independently, hydrogen atom or C1-4 alkyl,
   R^{45D} is C1-4 alkyl,
   R^{46D} is hydrogen atom or C1-4 alkyl,
   R^{47D} is hydrogen atom or C1-4 alkyl,
   Cyc10^{D} is a C3-12 mono- or bi-carbocyclic ring or a 3- to 12-membered mono- or bi-heterocyclic ring substituted with 1-5 of substitutes of the following (a)-(j) or unsubstituted:
   (a) C1-4 alkyl, (b) C2-5 acyl, (c) 1-4 alkoxy, (d) halogen atom, (e) hydroxy, (f) nitro, (g) cyano, (h) amine, (i) CF₃, (j) OCF₃,
      W^{D} is -O-, -S(O)_{pD}- or -NR^{48D}-,
      R^{48D} is hydrogen atom or C1-4 alkyl.) and a non-toxic salt thereof.
(5) In the specification of WO99/47497, a compound represented by formula (E) 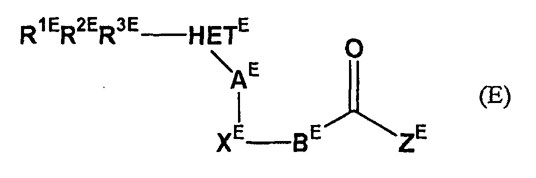 (wherein HET^{E} represents a 5-12 membered monocyclic or bicyclic aromatic ring system containing 0-3 heteroatoms selected from O, S(O)_{nE} and N(O)_{mE} wherein m^{E} is 0 or 1 and n^{E} is 0,1 or 2,
   A^{E} is a one or two atom moiety and is selected from the group consisting -W^{E}-, -C(O)-, -C(R^{7E})-W^{E}-, -W^{E}-C(R^{7E})₂-, -CR^{7E}(OR^{20E})-, -C(R^{7E})₂-, -C(R^{7E})₂-C(OR^{20E})R^{7E}-, -C(R^{7E})₂-C(R^{7E})₂- or -CR^{7E}=CR^{7E}-, wherein W^{E} represents O, S(O)_{nE} or NR^{17E},
   X^{E} represents a 5-10 membered monocyclic or bicyclic aryl or a 5-10 membered monocyclic or bicyclic heteroaryl having 1-3 heteroatoms selected from O, S(O)_{nE} and N(O)_{mE}, and optionally substituted with R^{14E} or R^{15E}, and A^{E} and B^{E} bind to the ortho position of aryl or heteroaryl,
   Y^{E} represents O, S(O)_{nE}, NR^{17E}, a bond or -CR^{18E}=CR^{18E}-,
   B^{E} represents -(C(R^{18E})₂)_{pE}-Y^{E}-(C(R^{18E})₂)_{qE}, p^{E} and q^{E} are independently 0-3, such that when Y^{E} represents O, S(O)_{nE}, NR^{17E} or -CR^{18E} =CR^{18E}-, p^{E} + q^{E} is 0-6, and when Y^{E} represents a bond, then p^{E} + q^{E} is 1-6,
   Z^{E} is OH or NHSO₂R^{19E},
   R^{1E}, R^{2E} and R^{3E} independently represent H, halogen atom, lower alkyl, lower alkenyl, lower alkynyl, lower alkenyl-HET^{E}(R^{aE})₄₋₉-, -(C(CR^{4E})₂)_{pE})SR^{5E}, -(C(R^{4E})₂)_{pE}OR^{8E}, -(C(R^{4E})₂)_{pE}N(R^{6E})₂, CN, NO₂, -(C(R^{4E})₂)_{pE}C(R^{7E})₃, -COOR^{9E}, -CON(R^{6E})₂ or -(C(R^{4E})₂)_{pE}S(O)_{nE}R^{10E},
   each R^{4E} is independently H, F, CF₃ or lower alkyl, or
   two R^{4E} groups are taken in conjunction and represent a ring of up to six atoms, optionally having one heteroatom selected from O, S(O)_{nE} and N(O)_{mE},
   each R^{5E} is independently lower alkyl, lower alkenyl, lower alkynyl, CF₃, lower alkyl-HET^{E}, lower alkenyl-HET^{E} or -(C(R^{18E})₂)_{pE}Ph(R^{11E})₀₋₂,
   each R^{6E} is independently H, lower alkyl, lower alkenyl, lower alkynyl, CF₃, Ph or Bn, or when two R^{6E} groups are attached to N, they may be taken in conjunction and represents a ring of up to 6 atoms, optionally having an additional heteroatom selected from O, S(O)_{nE} and N(O)_{mE},
   each R^{7E} is independently H, F, CF₃, lower alkyl, or two R^{7E} groups are taken in conjunction and represent an aromatic or aliphatic ring of 3 to 6 members having 0-2 heteroatoms selected from O, S(O)_{nE} and N(O)_{mE},
   each R^{8E} represents H or R^{5E},
   each R^{9E} is independently H, lower alkyl, lower alkenyl, lower alkynyl, Ph or Bn,
   each R^{10E} is independently lower alkyl, lower alkenyl, lower alkynyl, CF₃, Ph(R^{11E})₀₋₃, CH₂Ph(R^{11E})₀₋₃ or N(R^{6E})₂,
   each R^{11E} is independently lower alkyl, SR^{20E}, OR^{20E}, N(R^{6E})₂, -COOR^{12E}, -CON(R^{6E})₂, -COR^{12E}, CN, CF₃, NO₂ or halogen atom,
   each R^{12E} is independently H, lower alkyl or benzyl,
   each R^{13E} is independently H, halogen atom, lower alkyl, O-lower alkenyl, S-lower alkyl, N(R^{6E})₂, COOR^{12E}, CN, CF₃ or NO₂,
   R^{14E} and R^{15E} are independently lower alkyl, halogen atom, CF₃, OR^{16E}, S(O)_{nE}R^{16E} or C(R^{16E})₂OR^{17E},
   each R^{16E} is independently H, lower alkyl, lower alkenyl, Ph, Bn or CF₃,
   each R^{17E} is independently H, lower alkyl or Bn,
   each R^{18E} is independently H, F or lower alkyl, or two R^{18E} groups taken in conjunction and represent a ring of 3 to 6 members optionally containing one heteroatom selected from O, S(O)_{nE} and N,
   each R^{19E} is independently lower alkyl, lower alkenyl, lower alkynyl, CF₃, HET(R^{aE})₄₋₉, lower alkyl-HET(R^{aE})₄₋₉, lower alkenyl -HET(R^{aE})₄₋₉,
   each R^{20E} is independently H, lower alkyl, lower alkenyl, lower alkynyl, CF₃ or Ph(R^{13E})₂,
   each R^{aE} is independently selected from the group consisting of:
   H, OH, halogen atom, CN, NO₂, amino, C1-6 alkyl, C2-6alkenyl, C2-6alkynyl, C1-6 alkoxy, C2-6 alkenyloxy, C2-6 alkynyloxy, C1-6 alkylamino, di(C1-6 alkyl)amino, CF₃, C(O)C1-6 alkyl, C(O)C2-6alkenyl, C(O)C2-6alkynyl, COOH, COO(C1-6)alkyl, COO(C2-6)alkenyl and COO(C2-6)alkynyl;
   said alkyl, alkenyl, alkynyl or the alkyl portions of alkylamino or dialkylamino being optionally substituted with 1-3 of;
   OH, halogen atom, aryl, C1-6 alkoxy, C2-6 alkenyloxy, C2-6 alkynyloxy, CF₃, CO(C1-6)alkyl, CO(C2-6)alkenyl, CO(C2-6)alkynyl, COOH, COO(C1-6)alkyl, COO(C2-6)alkenyl, COO(C2-6)alkynyl, NH₂, NH(C1-6)alkyl and N(C1-6 alkyl)₂) or a non-toxic salt thereof.
(6) In the specification of WO00/20371, a compound represented by formula (F)

   Ar^{1F}-W^{F}-Ar^{2F}-X^{F}-Q^{F} (F)

   (wherein Ar^{1F} is an aryl or heteroaryl group, optionally substituted with R^{1F} or R^{3F},
   R^{1F} is Y^{F}_{mF}-R^{2F}, Y^{F}_{mF}-Ar^{3F}, halogen atom, N(R^{5F})₂, CN, NO₂, C(R^{6F})₃, CON(R^{5F})₂, S(O)_{nF}R^{7F} or OH,
   Y^{F} represents a linker between R^{2F} or Ar^{3F} and Ar^{1F} containing 0-4 carbon atoms and not more than one heteroatom selected from O, N and S, said linker optionally containing CO, S(O)_{nF}, -C=C- or an acetylenic group, and said linker being optionally substituted by R^{2F},
   m^{F} is 0 or 1,
   n^{F} is 0,1 or 2,
   R^{2F} represents H, F, CHF₂, CF₃, lower alkyl or hydroxyl(C1-6)alkyl, or two R^{2F} groups may be joined together and represent a carbocyclic ring of up to six members, said ring containing not more than one heteroatom selected from O, N and S,
   Ar^{3F} represents an aryl or heteroaryl group, optionally substituted with R^{3F};
   R^{3F} is R^{4F}, halogen atom, halo(C1-6)alkyl, N(R^{5F})₂, CN, NO₂, C(R^{6F})₃, CON(R^{5F})₂, OR^{4F}, SR^{4F} or S(O)_{nF}R^{7F},
   R^{4F} is H, lower alkyl, lower alkenyl, lower alkynyl, CHF₂ or CF₃,
   R^{5F} is R^{4F}, Ph or Bn, or two R^{5F} groups in combination with the atom to which they are attached represent a ring of up to 6 members containing carbon atoms and up to 2 heteroatoms selected from O, N and S,
   R^{6F} is H, F, CF₃ or lower alkyl, or two R^{6F} groups may be taken together and represent a ring of up to 6 members containing carbon atoms and 0-2 heteroatoms selected from O, N and S,
   R^{7F} is lower alkyl, lower alkenyl, lower alkynyl, CHF₂, CF₃, N(R^{5F})₂, Ph(R^{8F})₂ or CH₂Ph(R^{8F})₂,
   R^{8F} is R^{4F}, OR^{4F}, SR^{4F} or halogen atom,
   W^{F} represents a 3-6 membered linking group containing 0 to 2 heteroatoms selected from O, N and S, said linking group optionally containing CO, S(O)_{mF}, C=C or an acetylenic group, and optionally being substituted with R^{9F},
   R^{9F} is R^{2F}, lower alkenyl, lower alkynyl, OR^{4F} or SR^{4F},
   Ar^{2F} represents an aryl or heteroaryl group, optionally substituted with R^{3F},
   R^{10F} represents R^{4F}, halogen atom, N(R^{5F})₂, CN, NO₂, C(R^{6F})₃, OR^{4F}, SR^{4F} or S(O)_{nF}R^{7F},
   X^{F} represents a linker which is attached to Ar^{2F} ortho to the attachment of W^{F}, said linker containing 0-4 carbon atoms and not more than one heteroatom selected from O, N and S, said linker further optionally containing CO, S(O)_{nF}, C=C or an acetylenic group, and said linker being optionally substituted with R^{11F},
   R^{11F} is R^{9F},
   Q^{F} represents a group selected from COOH, tetrazole, SO₃H, hydroxamic acid, CONHSO₂R^{12F} and SO₂NHCOR^{12F},
   R^{12F} represents a group selected from CF₃, lower alkyl, lower alkenyl, lower alkynyl and Z^{F}Ar^{4F},
   Z^{F} is a linker containing 0-4 carbon atoms, optionally substituted with R^{13F},
   R^{13F} is R^{9F},
   Ar^{4F} is an aryl or heteroaryl group optionally substituted with R^{14F} and
   R^{14F} is R^{10F} or NHCOMe.) or a non-toxic salt thereof.
(7) In the specification of W02001/19814, a compound represented by formula (G) 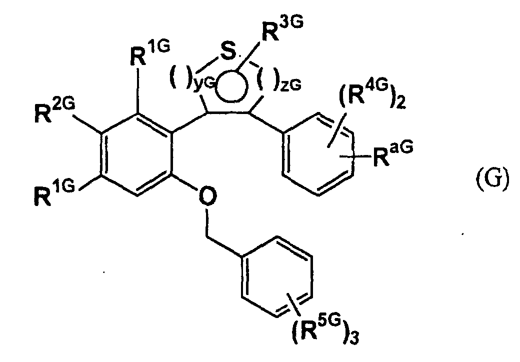 (wherein y^{G} and z^{G} are independently 0-2, wherein y^{G}+z^{G}=2,
   R^{aG} is selected from the group consisting of:
   1) heteroaryl, wherein heteroaryl is selected from the group consisting of:
      a) furyl, b) diazinyl, triazinyl or tetrazinyl, c) imidazolyl, d) isoxazolyl,
      e) isothiazolyl, f)oxadiazolyl, g) oxazolyl, h)pyrazolyl, i)pyrrolyl, j) thiadiazolyl, k) thiazolyl, l) thienyl, m) triazolyl and n) tetrazolyl; wherein heteroaryl is optionally substituted with one or more substituents selected from R^{11G} or C1-4 alkyl;
   2) -COR^{6G},
   3) -NR^{7G}R^{8G},
   4) -SO₂R^{9G},
   5) hydroxy,
   6) C1-6 alkoxy, optionally substituted with one or more substituents selected from R^{11G} and
   7) C1-6 alkyl, C2-6 alkenyl or C3-6 cycloalkyl, optionally substituted with one or more substituents selected from R^{11G}, and further optionally substituted with 1-3 substituents selected from the group consisting of:
      (a) -COR^{6G}, (b) -NR^{7G}R^{BG}, (c) -SO₂R^{9G}, (d) hydroxyl, (e) C1-6 alkoxy or haloC1-6 alkoxy and (f) heteroaryl;
   wherein R^{aG} is positioned on the phenyl ring to which it is bonded in a 1,3 or 1,4 relationship relative to the thienyl group represented in formula (G); each R^{1G}, R^{2G}, R^{3G}, R^{4G} and R^{5G} are independently selected from the group consisting of:
   1) hydrogen atom,
   2) halogen atom,
   3) C1-6 alkyl,
   4) Cl-6 alkoxy,
   5) Cl-6 alkylthio,
   6) nitro,
   7) carboxy and
   8) CN
   wherein items (3)-(5) above are optionally substituted with one or more R^{11G},
   R^{6G} is selected from the group consisting of hydrogen, hydroxy, C1-6 alkyl, C1-6 alkoxy and NR^{7G}R^{8G}, wherein C1-6 alkyl or C1-6 alkoxy are optionally substituted with one or more R^{11G},
   R^{7G} and R^{8G} are independently selected from the group consisting of:
   1) hydrogen atom,
   2) hydroxy,
   3) SO₂R^{9G},
   4) C1-6 alkyl,
   5) C1-6 alkoxy,
   6) phenyl,
   7) naphthyl,
   8) furyl,
   9) thienyl and
   10) pyridyl, wherein items (4)-(5) above are optionally substituted with one or more R^{11G}, and items (6)-(10) above are optionally substituted with one or more substituents selected from R^{11G} and C1-4 alkyl,
   R^{9G} is selected from the group consisting of
   1) hydroxyl,
   2) N(R^{10G})₂,
   3) C1-6 alkyl optionally substituted with one or more R^{11G},
   4) phenyl,
   5) naphthyl,
   6) furyl,
   7) thienyl and
   8) pyridyl,
   wherein items (4)-(8) above are optionally substituted with one or more substituents independently selected from R^{11G} and C1-4 alkyl,
   R^{10G} is hydrogen atom or C1-6 alkyl and
   R^{11G} is halogen atom, hydroxyl, C1-3 alkoxy, nitro, N(R^{10G})₂ or pyridyl.) or a pharmaceutically acceptable salt, hydrate or ester thereof.
(8) In the specification of WO2001/19819, a compound represented by formula (H) 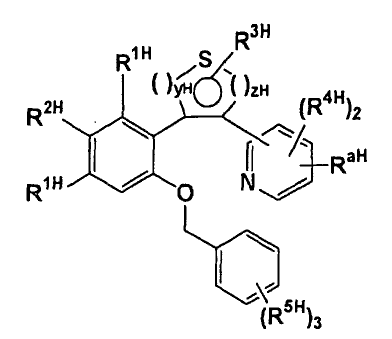 (wherein: y^{H} and z^{H} are independently 0-2, such that y^{H}+z^{H}=2,
   R^{aH} is selected from the group consisting of:
   1) heteroaryl, wherein heteroaryl is selected from the group consisting of:
      a) furyl, b) diazinyl, triazinyl or tetrazinyl, c) imidazolyl, d) isoxazolyl,
      e) isothiazolyl, f) oxadiazolyl, g) oxazolyl, h) pyrazolyl, i) pyrrolyl, j) thiadiazlolyl, k) thiazolyl, l) thienyl, m) triazolyl and n) tetrazolyl; wherein heteroaryl is optionally substituted with 1-3 substituents independently selected from R^{11G} and C1-4 alkyl:
   2) -COR^{6H},
   3) -NR^{7H}R^{8H},
   4) -SO₂R^{9H},
   5) hydroxy,
   6) C1-6 alkoxy, optionally substituted with 1-3 of R^{11H} and
   7) C1-6 alkyl, C2-6 alkenyl or C3-6 cycloalkyl, optionally substituted with 1-3 of R^{11H}, and further optionally substituted with 1-3 substituents selected from the group consisting of:
      (a) -COR^{6H}, (b) -NR^{7H}R^{8H}, (c) -SO₂R^{9H}, (d) hydroxy, (e) C1-6 alkoxy or haloC1-6 alkoxy and (f) heteroaryl,
   such that R^{aH} is positioned on the pyridyl ring to which it is bonded in a 1,3 or 1,4 relationship relative to the thienyl group represented in formula (H),
   R^{1H}, R^{2H}, R^{3H}, R^{4H} and R^{5H} are independently selected from the group consisting of:
   1) hydrogen atom,
   2) halogen atom,
   3) C1-6 alkyl,
   4) C1-6 alkoxy,
   5) C1-6 alkylthio,
   6) nitro,
   7) carboxy and
   8) CN,
   wherein items (3)-(5) above are optionally substituted with one or more R^{11H},
   R^{6H} is selected from the group consisting of hydrogen atom, hydroxy, C1-6 alkyl, C1-6 alkoxy and NR^{7H}R^{8H}, wherein C1-6 alkyl or C1-6 alkoxy are optionally substituted with 1-3 substituents independently selected from R^{11H},
   R^{7H} and R^{8H} are independently selected from the group consisting of:
   1) hydrogen atom,
   2) hydroxy,
   3) SO₂R^{9H},
   4) C1-6 alkyl,
   5) C1-6 alkoxy,
   6) phenyl,
   7) naphthyl,
   8) furyl,
   9) thienyl and
   10) pyridyl,
   wherein items (4)-(5) above are optionally substituted with 1-3 of R^{11H}, and items (6)-(10) above are optionally substituted with 1-3 substituents independently selected from R^{11H} and C1-4 alkyl,
   R^{9H} is selected from the group consisting of
   1) hydroxy,
   2) N(R^{10H})₂,
   3) C1-6 alkyl, optionally substituted with 1-3 of R^{11H},
   4) phenyl,
   5) naphthyl,
   6) furyl,
   7) thienyl and
   8) pyridyl,
   wherein items(4)-(8) above are optionally substituted with 1-3 substituents independently selected from R^{11H} and C1-4 alkyl,
   R^{10H} is hydrogen atom or C1-6 alkyl,
   R^{11H} is selected from the group consisting of: halogen atom, hydroxy, C1-3 alkoxy, nitro, N(R^{10H})₂ and pyridyl.) or a pharmaceutically acceptable salt, hydrate or ester thereof.

The compounds represented by above formula (A) to (H) may be converted into a corresponding salt by known methods. Non-toxic and water-soluble salts are preferable.

Salts are salts of alkali metals, such as potassium, sodium, *etc*.; salts of alkaline-earth metals, such as calcium, magnesium, *etc*.; ammonium salts, pharmaceutically acceptable organic amines, such as tetramethylammonium, triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, *etc.*

Preferable acid addition salts are non-toxic and water-soluble salts. Acid addition salts are salts of inorganic acids such as hydrochloride, hydrobromide, sulfate, phosphate, nitrate; salts of organic acids e.g. acetate, trifluoroacetate, lactate, tartrate, oxalate, fumarate, maleate, citrate, benzoate, methanesulphonate, ethanesulphonate, benzenesulphonate, toluenesulphonate, isethionate, glucuronate, gluconate.

The compound of the present invention and a salt thereof may be converted into the corresponding hydrates by conventional means.

The specific compounds in the present invention are specific compounds described in the specifications of WO01/62708, WO02/16311, W002/20462, PCT/JP02/08120, WO99/47497, WO00/20371, WO2001/19814 or W02001/19819, for example, the compounds described in examples.

Preferably, it is the compounds which bind to and antagonize EP₃ receptor among the compound indicated in the above specification, and more preferably, it is the compounds which bind to and antagonize EP₃ receptor, specifically.

### [Toxicity]

The compound used in the present invention has low toxicity so that use of it as a pharmaceutical can be considered as safe enough.

### Industrial Applicability

### [Application to pharmaceuticals]

The compounds with antagonistic activity for EP₃ are useful in preventing and /or treating pruritus for mammal, especially human. More specially, pruritus is the disease with itch and the compounds are useful in preventing and/or treating pruritus by eczema, urticaria, contact dermatitis, atopic dermatitis, dermatitis herpetiformis, psoriasis, lichen planus, rhus dermatitis, biliary obstruction, uremia, lymphoma, leukemia, polycythemia vera, dry skin, or hemodialysis performed in treating renal involvement with chronic glomerulonephritis, diabetes mellitus, nephrosclerosis, cystic kidney or systemic disease, or conjunctivitis such as seasonal allergic conjunctivitis, acute conjunctivitis, chronic conjunctivitis, trachoma, perennial allergic conjunctivitis or spring catarrh etc.

The compounds represented by formula (I) or non-toxic salts thereof may be combined with other drugs and administered as combination drugs for:
(1) complementing and/or enhancing the preventive and/or therapeutic effects of the compounds;
(2) improving the dynamics and absorption of the compounds and reducing the administration dose thereof; and/or
(3) mitigation of the side effects of the compounds.

A combination drug of the compound of formula (I) with other drug may be administered in the form of a blend containing both of the components in a single preparation. Alternatively, the components may be processed into separate preparations and administered. The separate preparations may be administered either at the same time or at a definite time interval. In the case of administering at a definite time interval, the compound of formula (I) may be administered first followed by the administration of the other drug. Alternatively, the other drug may be administered first followed by the administration of the compound of formula (I). The administration methods may be either the same or different.

Diseases on which the preventive and/or therapeutic effects are exerted by the combination drug are not particularly restricted. That is, they may be any diseases so long as the preventive and/or therapeutic effects of the compounds of formula (I) thereon can be complemented and/or enhanced.

Examples of other drugs for complementing and/or enhancing the preventive and/or therapeutic effect of the compounds of formula (I) on pruritus include steroids, nonsteroid antiinflammatory drugs, immunosuppressants, mediator release inhibitors, leukotriene receptor antagonists, antihistamines, antiserotonins, other antiallergic agents, forskolin preparations, phosphodiesterase inhibitors, nitrogen monoxide synthase inhibitors, cannabinoide-2 receptor stimulating agents and the like.

Examples of the nonsteroid antiinflammatory drugs include sasapyrine, sodium salicylate, aspirin, aspirin dialuminate blend, diflunisal, indomethacin, sprofen, ufenamate, dimethylisopropylazulene, bufexamac, felbinac, diclofenac, tolmetin sodium, clinoril, fenbufen, nabumetone, proglumetacin, indometacin farnesil, acemetacin, proglumetacin maleate, amfenac sodium, mofezolac, etodolac, ibuprofen, ibuprofen piconol, naproxen, flurbiprofen, flurbiprofen axetil, ketoprofen, fenoprofen calcium, tiaprofen, oxaprozin, pyranoprofen, loxoprofen sodium, alminoprofen, zaltoprofen, mefenamic acid, aluminum mefenamate, tolfenamic acid, floctafenine, ketophenylbutazone, oxyfenbutazone, piroxicam, tenoxicam, ampiroxicam, napageln ointment, epirizol, tiaramide hydrochloride, tinoridine hydrochloride, emorfazone, sulpirin, migrenin, saridon, sedes G, amipylo N, sorbon, pyrazolone-based remedies for cold, acetoaminophen, fenacetine, dimethothiazine mesylate, meloxicam, celecoxib, rofecoxib, valdecoxib, simetride-containing agents, pyrazolone-free remedies for cold and the like.

Examples of the steroids include, e.g., as drugs for external use, clobetasol propionate, diflorasone acetate, fluocinonide, mometazone furancarboxylate, betametazone dipropionate, betametazone butyrate propionate, betametazone valerate, difluprednate, budesonide, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone valerate acetate, fluocinolone acetonide, beclometasone propionate, triamcinolone acetonide, flumetasone pivalate, alclometasone propionate, clobetasone butyrate, prednisolone, beclomethasone propionate, fludroxycortide and the like.

Examples of drugs for internal use and injections include cortisone acetate, hydrocortisone, hydrocortisone sodium phosphate, hydrocortisone sodium succinate, fludrocortisone acetate, prednisolone, prednisolone acetate, prednisolone sodium succinate, prednisolone butyl acetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone sodium phosphate, dexamethasone palmitate, paramethasone acetate, betamethasone and the like.

Examples of inhalations include beclometasone propionate, fluticasone propionate, budesonide, flunisolide, triamcinolone, ST-126P, ciclesonide, dexamethasone palomithionate, mometasone furoate, prasterone sulfonate, deflazacort, methylprednisolon sleptanate, methylprednisolon sodium succinate and the like.

Examples of the immune suppressants include protopic (FK-506), methotrexate, cyclosporin, ascomycin, leflunomide, bucillamine, salazosulfapyridine and the like.

Examples of the mediator release inhibitors include tranilast, sodium cromoglycate, amlexanox, repirinast, ibudilast, tazanolast, pemirolast potassium and the like.

Examples of the leukotriene receptor antagonists include pranlukast hydrate, montelukast, zafirlukast, MCC-847, KCA-757, CS-615, YM-158, L-740515, CP-195494, LM-1484, RS-635, A-93178, S-36496, BIIL-284, ONO-4057 and the like.

Examples of the antihistamines include ketotifen fumarate, mequitazine, azelastine hydrochloride, oxatomide, terfenadine, emedastine fumarate, epinastine hydrochloride, astemizole, ebastine, cetirizine hydrochloride, bepotastine, fexofenadine hydrochloride, loratadine, desloratadine, olopatadine hydrochloride, clemastine fumarate, chloropheniramine maleate, cyproheptadine hydrochloride, promethazine hydrochloride, homochlorcyclizine hydrochloride, Diphenhydramine Hydrochloride, TAK-427, ZCR-2060, NIP-530, mometasone furoate, mizolastine, BP-294, andolast, auranofin, acrivastine, *etc*.

Examples of the other antiallergic agents include suplatast tosilate, ozagrel hydrochloride, seratrodast, ramatroban, etc.

Examples of the phosphodiesterase inhibitors include PDE4 inhibitors such as roliplam, cilomilast, Bay 19-8004, NIK-616, roflumilast (BY-217), cipamfylline (BRL-61063), atizoram (CP-80633), SCH-351591, YM-976, V-11294A, PD-168787, D-4396, IC-485 and the like.

The ratio by mass of the compounds of formula (I) to other drugs is not particularly limited.

Two or more of other drugs optionally selected can be used in combination.

Other drugs to be used for complementing and/or enhancing the preventive and/or therapeutic effects of the compounds of formula (I) involve not only those which have been found out hitherto based on the above-described mechanism but also those which will found out in future.

To employ the compounds with EP₃ antagonistic activity, non-toxic salts, acid addition salts or hydrate thereof for the above-described purposes, they are usually administered systemically or topically, and orally or parenterally.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 0.001 mg to 100 mg, by parenteral administration, up to several times per day. Preferably, the doses are from 0.1 mg to 100 mg as preparations for external use or from 0.001 mg to 1 mg as eye drops, up to several times per day.

As mentioned above, the doses to be used depend upon various conditions. Therefore, there are cases in which doses lower than or greater than the ranges specified above may be used.

To administrate the compounds in the present invention, use is made of solid preparations for internal use and liquid preparations for internal use for oral administration as well as preparations for external use, eye drops, injections, suppositories and the like for parenteral administration.

Examples of the solid preparations for internal use for oral administration include tablets, pills, capsules, powders, granules and the like. The capsules include hard capsules and soft capsules.

Such a solid preparation for internal use is prepared by a formulation method commonly employed by using one or more active substances either as such or as a mixture with an excipient (lactose, mannitol, glucose, microcrystalline cellulose, starch, *etc*.), a binder (hydroxypropylcellulose, polyvinylpyrrolidone, magnesium metasilicate aluminate, *etc*.), a disintegrating agent (calcium cellulose glycolate, *etc*.), a lubricant (magnesium stearate, *etc*.), a stabilizer, and a dissolution aid (glutamic acid, aspartic acid, *etc*.). If necessary, it may be coated with a coating agent (sucrose, gelatin, hydroxypropylcellulose, hydroxypropylmethylcellulose phthalate, *etc.*). It may be coated with two or more layers. Moreover, capsules made of an absorbable material such as gelatin are involved in the scope thereof.

The liquid preparations for internal use for oral administration involve pharmaceutically acceptable solutions, suspensions, emulsions, syrups, elixirs and the like. Such a liquid preparation is prepared by dissolving, suspending or emulsifying one or more active substances in a diluent commonly employed (purified water, ethanol, a mixture thereof, *etc.*). Besides such liquid forms may also comprise some additives, such as humectants, suspending agents, emulsifying agents, sweetening agents, flavoring agents, aroma, preservative or buffering agents etc.

The dosage forms of the parenteral administration preparations for external use involve ointments, gels, creams, fomentations, patches, liniments, atomized agents, inhalations, sprays, aerosols, eye drops, nasal drops and the like. Such a preparation contains one or more active substances and is prepared by a publicly known method or in accordance with a formulation commonly employed.

Ointments are prepared in accordance with a publicly known formulation or a formulation commonly employed. For example, they are prepared by levigating or melting one or more active substances in a base. The ointment base is selected from among publicly known ones or those commonly employed. For example, use may be made of one base or a mixture of two or more thereof selected from higher fatty acids or higher fatty acid esters (adipic acid, myristic acid, palmitic acid, stearic acid, oleic acid, adipic acid esters, myristic acid esters, palmitic acid esters, stearic acid esters, oleic acid esters, *etc*.), waxes (beeswax, whale wax, ceresin, *etc*.), surfactants (polyoxyethylene alkyl ether phosphoric acid esters, *etc*.), higher alcohols (cetanol, stearyl alcohol, cetostearyl alcohol, *etc*.), silicone oils (dimethylpolysiloxane, *etc*.), hydrocarbons (hydrophilic vaseline, white vaseline, refined lanolin, liquid paraffin, *etc.*), glycols (ethylene glycol, diethylene glycol, propylene glycol, polyethylene glycol, macrogol, *etc*. ), vegetable oils (castor oil, olive oil, sesame oil, turpentine oil, *etc*.), animal oils (mink oil, yolk oil, squalane, squalene, *etc*.), water, absorption promoters and skin irritation inhibitors. The ointments may further contain a humectant, a preservative, a stabilizer, an antioxidant, a flavor, *etc*.

Gels are prepared in accordance with a publicly known formulation or a formulation commonly employed. For example, they are prepared by melting one or more active substances in a base. The gel base is selected from among publicly known ones or those commonly employed. For example, use may be made of one base or a mixture of two or more thereof selected from among lower alcohols (ethanol, isopropyl alcohol, *etc*.), gelling agents (carboxymethylcellulose, hydroxyethylcellulose, hydroxypropylcellulose, ethylcellulose, *etc*.), neutralizing agents (triethanolamine, diisopropanolamine, *etc*.), surfactants (polyethylene glycol monostearate, *etc*.), gums, water, absorption promoters and skin irritation inhibitors. The gels may further contain a preservative, an antioxidant, a flavor, etc.

Creams are prepared in accordance with a publicly known formulation or a formulation commonly employed. For example, they are prepared by melting or emulsifying one or more active substances in a base. The cream base is selected from among publicly known ones or those commonly employed. For example, use may be made of one base or a mixture of two or more thereof selected from among higher fatty acid esters, lower alcohols, hydrocarbons, polyhydric alcohols (propylene glycol, 1,3-butylene glycol, *etc*.), higher alcohols (2-hexyldecanol, cetanol, *etc*.), emulsifiers (polyoxyethylene alkyl ethers, fatty acid esters, *etc*.), water, absorption promoters and skin irritation inhibitors. The creams may further contain a preservative, an antioxidant, a flavor, etc.

Fomentations are prepared in accordance with a publicly known formulation or a formulation commonly employed. For example, they are prepared by melting one or more active substances in a base, kneading and then applying and spreading the kneaded matter on a substrate. The fomentation base is selected from among publicly known ones or those commonly employed. For example, use may be made of one base or a mixture of two or more thereof selected from among thickeners (polyacrylic acid, polyvinylpyrrolidone, gum acacia, starch, gelatin, methylcellulose, *etc.*), moistening agents (urea, glycerin, propylene glycol, *etc.*), fillers (kaolin, zinc oxide, talc, calcium, magnesium, *etc.*), water, dissolution aids, tackifiers and skin irritation inhibitors. The fomentations may further contain a preservative, an antioxidant, a flavor, *etc*.

Patches are prepared in accordance with a publicly known formulation or a formulation commonly employed. For example, they are prepared by melting one or more active substances in a base and then applying and spreading on a substrate. The patch base is selected from among publicly known ones or those commonly employed. For example, use may be made of one base or a mixture of two or more thereof selected from among polymer bases, fats and oils, higher fatty acids, tackifiers and skin irritation inhibitors. The patches may further contain a preservative, an antioxidant, a flavor, etc.

Liniments are prepared in accordance with a publicly known formulation or a formulation commonly employed. For example, they are prepared by dissolving, suspending or emulsifying one or more active substances in one or more media selected from among water, alcohols (ethanol, polyethylene glycol, *etc.*), higher fatty acids, glycerin, soap, emulsifiers, suspending agents and the like. The liniments may further contain a preservative, an antioxidant, a flavor, *etc*.

Atomized agents, inhalations and sprays may contain, in addition to a diluent commonly employed, a stabilizer such as sodium hydrogen sulfite, a buffer for imparting isotonicity, for example, an isotonic agent such as sodium chloride, sodium citrate or citric acid. Methods for producing a spray are described in detail in, for example, U.S. Patent 2,868,691 and U.S. Patent 3,095,355. Moreover, it is used aerosol agents.

Eye drops for parenteral administration may be in the form of liquid, suspension, emulsion, liquid dissolved in a solvent in use or ointment.

These eye drops are prepared by any known method. For example, one or more active substances are dissolved, suspended or emulsified in a solvent. As such a solvent for eye drops there may be used sterilized purified water, physiological saline and other aqueous or nonaqueous solvents (e.g., vegetable oil), singly or in combination thereof. The eye drops may contain one or more solvent optionally selected from an isotonic agent (e.g., sodium chloride, concentrated glycerin), a buffering agent (e.g., sodium phosphate, sodium acetate), a surfactants (e.g., Polysolvate 80 (trade name), polyoxyl stearate 40, polyoxyethylene-hardened castor oil), a stabilizer (sodium citrate, sodium edetate), a preservative (e.g., benzalconium chloride, paraben), *etc.* The eye drops are sterilized at the final step or prepared by an aseptic process. Alternatively, an aseptic solid agent such as freeze-dried product which has previously been prepared may be rendered aseptic or dissolved in an aseptic distilled water for injection or other solvent before use.

The injections for parenteral administration involve solutions, suspensions, emulsions and solid injections to be dissolved or suspended before use. Such an injection is used by dissolving, suspending or emulsifying one or more active substances in a solvent. As a solvent, use may be made of, for example, distilled water for injection, physiological saline, vegetable oils, alcohols such as propylene glycol, polyethylene glycol or ethanol and mixtures thereof. The injection may further contain a stabilizer, a dissolution aid (glutamic acid, aspartic acid, Polysorbate 80 (registered trade name), *etc*.), a suspending agent, an emulsifier, a soothing agent, a buffer, a preservative, *etc*. Such an injection may be produced by sterilizing at the final step or employing aseptic process. Alternatively, it is also possible that an aseptic solid product such as a freeze-dried product is produced and sterilized or dissolved in aseptic distilled water for injection or another solvent before use.

The inhalations for parenteral administration involve aerosols, powders for inhalation and liquids for inhalation. Such inhalations may be in the form to be dissolved or suspended in water or another adequate medium before use.

The inhalations may be prepared in accordance with a publicly known method.

For example, liquid preparations for inhalation may be, if, necessary, prepared by appropriately selecting a preservative (benzalkonium chloride, paraben, *etc*.), a colorant, a buffer (sodium phosphate, sodium acetate, *etc*.), an isotonic agent (sodium chloride, concentrated glycerin, *etc*.), a thickener (carboxyvinyl polymer, *etc.*), an absorption promoter and the like.

Powders for inhalation may be prepared, if necessary, by appropriately selecting a lubricant (stearic acid, its salt, *etc*.), a binder (starch, dextrin, *etc*.), an excipient (lactose, cellulose, *etc*.), a colorant, a preservative (benzalkonium chloride, paraben, *etc.*), an absorption promoter, etc.

When the liquids for inhalation are administered, a sprayer (atomizer, nebulizer) is usually used. When the powders for inhalation are used, an inhalation administration apparatus for powder agents is usually used.

Other compositions for parenteral administration include suppositories and pessaries for vaginal administration which contain one or more active substances and are prepared in accordance with common formulations.

### Brief description of the drawings

Figure 1 is the graph that shows the number of scratching in serotonin and PGE₂-induced pruritus when the compound (1) and the compound (2) were administered.
Figure 2 is the graph that shows the number of scratching in serotonin and PGE₂-induced pruritus when the compound (3) was administered.
Figure 3 is the graph that shows the number of scratching in allergic dermatitis model when the compound (4) was administered.

### Best Mode for Carrying Out the Invention

The effect for pruritus of the compounds having antagonistic activity for EP₃ receptor was shown by the following experiments. However, that the present invention is not limited thereto.

### Example 1 : The inhibitory effect against pruritus induced by serotonin and PGE₂.

The following compounds having antagonistic activity for EP₃ receptor were used as test compounds
- Compound (1):: sodium 3-(2-(2-(naphthalen-2-yl)ethoxy)-4-(pyrazol-1-ylmethyl)phenyl)propanoate (dose : 100 mg/5 ml/kg) [The compound described in Example 3(12) in the specification of PCT/JP02/08120],
- Compound (2) :: sodium 2-[4-cyano-2-[4-methyl-2-(1-naphthyl)pentanoylamino]phenylmethyl]benzoate (dose: 100mg/5ml/kg) [The compound described in Example 4(18) in the specification of WO01/62708],
- Compound (3) :: (2E)-N-(5-bromo-2-methoxyphenylsulfonyl)-3-(2-(naphthalen-2-ylmethyl)phenyl)-2-propenamide (dose : 300 mg/5 ml/kg) [The compound described as Compound 301 in the specification of WO99/47497].

The back of male ICR mice (Charles River Japan Inc., 5 week-old) were sheared by hair clipper. 0.5% MC or the above test compound (100 mg/5 ml/kg or 300 mg/5 ml/kg) was administered orally. After 15 minutes or 1 hour, serotonin (3 µg) or mixed solution of serotonin (3 µg) and PGE₂ (1 nmol) was injected intradermally (20 µl) into the back of mouse. Scratching of the injected site or the perimeter by the behind paws of mouse was characterized as itch. The scratching was counted for 30 minutes from the injection.

### Group configuration-1

- Vehicle group:: 0.5 % MC [administration at 15 minutes before the induction]/serotonin (n=10)
- Control group :: 0.5 % MC [administration at 15 minutes before the induction]/serotonin+PGE₂ (n=10)
- Test compounds group :: compound (1) [administration at 15 minutes before the induction]/serotonin+PGE₂ (n=10)
compound (2) [administration at 15 minutes before the induction]/serotonin+PGE₂ (n=10)

### Group configuration-2

- Vehicle group :: 0.5 % MC [administration at 1 hour before the induction]/serotonin (n=10)
- Control group :: 0.5 % MC [administration at 1 hour before the induction]/serotonin+PGE₂ (n=10)
- Test compounds group:: compound (3) [administration at 1 hour before the induction]/serotonin+PGE₂ (n=10)

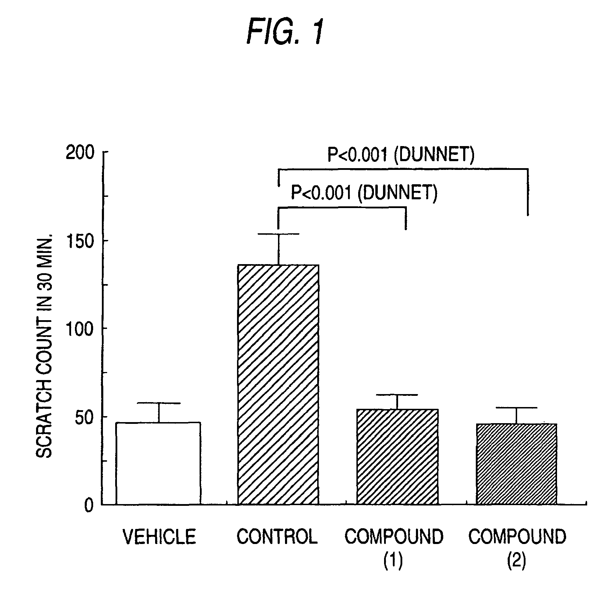

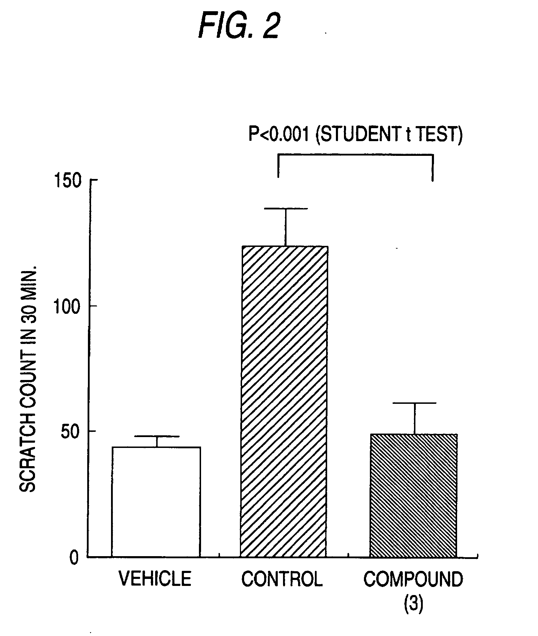

The result was showed in the figure 1 and the figure 2. The figure 1 suggests that in the model of pruritus induced by serotonin and PGE₂, the compound (1) (100mg/5ml/kg) and the compound (2) (100mg/5ml/kg) inhibited significantly against control, and the figure 2 suggests in the model of pruritus induced by serotonin and PGE₂, the compound (3) (300 mg/5 ml/kg) inhibited significantly against control.

### Example 2 : The inhibitory effect against pruritus in a model of allergic dermatitis.

- Compound (4) :: 3-(2-(2-(naphthalen-2-yl)ethoxy)-4-(pyrazol-1-ylmethyl)phenyl)propanoic acid [The compound described in Example 3(12) in the specification of PCT/JP02/08120] was used as a test compound.

The back of male BALB/c mice (Charles River Japan Inc., 6 week-old) were sheared by hair clipper. 0.15% dinitrofluorobenzene (DNFB) dissolved in mixed solution of acetone and olive oil as hapten was administered 50 µl by micro pipette in the back once a week. After forth applications, the scratching was measured from 3 to 5 hours and from 24 to 26 hours and mice were divided to each group referring the scratch data. 0.5% MC or the test compound (100 mg/5 ml/kg) was administered orally forth every 30 minutes from 30 minutes before 3 and 24 hours, respectively after fifth application of hapten. The scratching was videotaped from 3 to 5 hours and from 24 to 26 hours, respectively, after fifth application of hapten. The scratching was counted and estimated.

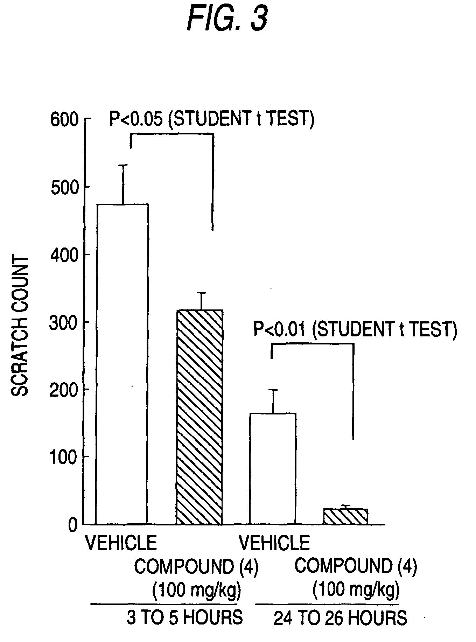

The result was showed in the figure 3. The figure 3 suggests that in the model of allergic dermatitis, the compound (4) (100 mg/5 ml/kg) inhibited significantly against control.

In the above Example 1 and 2, it was proved that the compounds with antagonistic activity for EP₃ receptor have significant inhibitory effect in the both models of pruritus. These were revealed for the first time.

### Preparation Example 1

The following components were admixed in a conventional method, punched out to give 100 tablets each containing 50 mg of active ingredient.
· sodium 3-(2-(2-(naphthalen-2-yl)ethoxy)-4-(pyrazol-1-ylmethyl)phenyl)propanate 5.0 g
· calcium carboxymethylcellulose (disintegrant) 0.2 g
· magnesium stearate (lubricant) 0.1 g
· microcrystalline cellulose 4.7 g

## Claims

1. An agent for treatment and/or prevention of pruritus which comprise, as the active ingredient, an antagonist to EP₃ which is one of prostaglandin E₂ receptor subtypes.

2. The agent for treatment and/or prevention of pruritus according to claim 1, wherein the antagonist to EP₃ is a compound selected from
(1) a compound described in the specification of WO01/62708,
(2) a compound described in the specification of WO02/16311,
(3) a compound described in the specification of WO02/20462,
(4) a compound described in the specification of PCT/JP02/08120,
(5) a compound described in the specification of WO99/47497,
(6) a compound described in the specification of WO00/20371,
(7) a compound described in the specification of WO2001/19814 and
(8) a compound described in the specification of WO2001/19819.

3. The agent for treatment and/or prevention of pruritus according to claim 1, wherein pruritus is eczema, urticaria, contact dermatitis, atopic dermatitis, dermatitis herpetiformis, psoriasis, lichen planus, rhus dermatitis, biliary obstruction, uremia, lymphoma, leukemia, polycythemia vera, dry skin, or hemodialysis performed in treating renal involvement with chronic glomerulonephritis, diabetes mellitus, nephrosclerosis, cystic kidney or systemic disease, or seasonal allergic conjunctivitis, acute conjunctivitis, chronic conjunctivitis, trachoma, perennial allergic conjunctivitis or spring catarrh.
